# EUROPEAN PATENT APPLICATION

(11) **EP 3 944 857 A1**
(43) Date of publication of application: **02.02.2022**
(21) Application number: 20188747.8
(22) Date of filing: 30.07.2020
(51) Int. Cl.: A61K 8/49, A61Q 5/00

(54) **USE OF PIROCTONE OLAMINE FOR REDUCING SHEDDING OF HAIR FIBRES**

(71) Applicant: Unilever Global IP Ltd, Wirral, CH62 4ZD (GB)
(72) Inventor: Bhogal, Ranjit Kaur, Shambrook, Bedfordshire MK44 1LQ (GB); Gunn, David Andrew, Shambrook, Bedfordshire MK44 1LQ (GB); Sawicka, Magdalena, Shambrook, Bedfordshire MK44 1LQ (GB)
(74) Representative: James, Helen Sarah

(57) **Abstract**

The invention provides the cosmetic use of piroctone olamine as an active ingredient for upregulating the expression of genes encoding cell adhesion molecules in cells of the hair follicle, and/or downregulating the expression of genes encoding proteases in cells of the hair follicle, thereby reducing shedding of hair club fibres from hair follicles.

## Description

### Field of the Invention

The invention relates to the use of piroctone olamine as an agent for reducing hair shedding.

### Background of the Invention

The hair follicle is a regenerating biological system whose primary function is to produce a hair fibre. The hair growth cycle consists of phases of active regeneration (anagen), apoptotic involution (catagen), and relative proliferative quiescence (telogen), which occur continuously throughout the follicle lifetime. Towards the end of catagen and at the beginning of telogen, a completely keratinized hair shaft with a characteristic depigmented and club-like base, termed the "hair club fibre", is held firmly by a specialized junction complex at the base of the bulge (a group of slow-cycling keratinocytes located below the sebaceous gland).

In humans, the hair club fibre is not usually retained through subsequent anagens, but shed from the follicle in a distinct and independent phase termed "exogen". Hair shedding is thus a natural consequence of the human hair cycle. However, the amount of hair that is shed depends on several internal and external factors, and the conscious perception of hair shedding can vary by individual. Premature or excessive hair shedding can be a preoccupation for many people, not only for those with a recognised hair loss disorder.

Most hair loss research has focused on the hair growth cycle, and less attention has been devoted to studying the mechanisms involved in exogen. Exogen is believed to be an active process and independent of telogen and anagen.

There is a continuing need for cosmetically acceptable treatments which are capable of reducing hair shedding through targeting of processes involved in exogen.

The present invention addresses this problem.

### Summary of the Invention

The invention provides the cosmetic use of piroctone olamine as an active ingredient for upregulating the expression of genes encoding cell adhesion molecules in cells of the hair follicle, and/or downregulating the expression of genes encoding proteases in cells of the hair follicle, thereby reducing shedding of hair club fibres from hair follicles.

The invention also provides piroctone olamine for use as an active ingredient for upregulating the expression of genes encoding cell adhesion molecules in cells of the hair follicle, and/or downregulating the expression of genes encoding proteases in cells of the hair follicle, thereby reducing shedding of hair club fibres from hair follicles.

The invention also provides the use of piroctone olamine as an active ingredient in, and for the manufacture of, topical compositions for upregulating the expression of genes encoding cell adhesion molecules in cells of the hair follicle, and/or downregulating the expression of genes encoding proteases in cells of the hair follicle, thereby reducing shedding of hair club fibres from hair follicles.

### Detailed Description and Preferred Embodiments

### Piroctone olamine

Piroctone olamine (1-hydroxy-4-methyl-6-(2,4,4-trimethyl)-2-(1H)pyridinone,2-aminoethanol salt) is an ethanolamine salt of the hydroxamic acid derivative piroctone. Piroctone olamine (also known as piroctone ethanolamine; is the ethanolamine salt of the hydroxamic acid derivative piroctone. It is known as an antimycotic agent (brand name Octopirox^{®}), and is used in antidandruff shampoos for the purpose of controlling the growth of *Malassezia spp.* It is believed to inhibit energy metabolism in mitochondria of pathogenic fungi by penetrating the fungal cell membrane and forming complexes with iron. The effect of piroctone olamine on the expression of genes encoding cell adhesion molecules and proteases in cells of the hair follicle has not been disclosed.

### Cell adhesion molecules

Cell adhesion molecules (or "CAMs") such as the desmosomal cadherins are considered to play an important role in anchoring the developing hair club fibre to its surrounding epithelial sac.

The desmosomal cadherins are a subfamily of the cadherin superfamily of CAMs, which is characterised by the presence of a variable number of successive extracellular cadherin (EC) repeat domains, each comprising c. 110 amino acids, that are rigidified by binding three Ca²⁺ ions at linker regions between these domains. These CAMs cooperate to make up the adhesive core of intercellular junctions known as desmosomes. In the human hair follicle, desmosomes are present in abundance between the trichilemmal keratin at the base of the hair club fibre and the surrounding epithelial sac.

Studies have shown that a loss of functional desmosomes in the innermost layer of cells surrounding the hair club fibre leads to splitting in the ORS, resulting in premature release of the hair club fibre from its surrounding epithelial sac.

A specific example of a CAM which is encoded by a gene whose expression can be upregulated by piroctone olamine in accordance with this invention is the desmosomal cadherin desmoglein-4. Desmoglein-4, which is encoded by DSG4, has been identified as an important desmosomal cadherin of the hair follicle. DSG4 is expressed in the precortex, keratinizing zone of the cortex, lower hair shaft cuticle, and the upper IRS cuticle. A mutation in DSG4 has been shown to lead to hair loss affecting the scalp, chest, arms, and legs.

Proteases are considered to play an important role in mediating the release of the hair club fibre from its surrounding epithelial sac.

Studies on hair club fibre extracts have demonstrated that the material surrounding the root contains significantly enhanced protease activity relative to that detected in the region distal to the root. Furthermore, spontaneously shed hair club fibres have been noted to lack cytological details, such as prominent nuclei and an abundant cytoplasm, which are seen in prematurely plucked club fibres. This indicates that the cells surrounding spontaneously shed hair club fibres have undergone proteolytic degradation.

Changes in protease expression in the interfollicular epidermis may affect the proteolytic balance in the hair follicle, leading to premature release of the hair club fibre from its surrounding epithelial sac.

Proteases which have been identified in the hair follicle and interfollicular epidermis include metalloproteases and serine proteases.
Specific examples of proteases which are encoded by genes whose expression can be downregulated by piroctone olamine in accordance with this invention are:

A disintegrin and metalloproteinase domain 8 (encoded by ADAM8), a zinc-dependent metalloproteinase which is a member of the ADAM (a disintegrin and metalloproteinase) family. ADAMs are typically found anchored to the cellular membrane and function as transmembrane proteases that act to cleave proteins from the cell surface. ADAMs appear to serve two generic functions: as 'sheddases' that release ectodomains of transmembrane-anchored cell surface proteins, and as proteases that can cleave extracellular matrix (ECM) molecules.

Serine protease 22 (encoded by PRSS22), which is a member of the tryptase family of proteases. PRSS22 is preferentially expressed by epithelial cells.

Human tissue kallikrein-related peptidases (KLKs) 6,8,11 and 13 (encoded by KLK6, KLK8, KLK11 and KLK13 respectively). KLKs are a family of extracellular serine proteases exhibiting trypsin-like or chymotrypsin-like activities. They have numerous putative extracellular matrix (ECM) substrates, and also show activity toward cell surface molecules. Collectively, this allows them to regulate the epidermal microenvironment. KLK6 and other KLKs have been implicated in the degradation of intercellular structures such as desmosomes, either directly or through participation in a proteolytic activation cascade

The piroctone olamine for use according to the invention is suitably formulated into a personal care composition which is suitable for topical application to the hair and scalp. Suitable personal care compositions for use in the invention include rinse-off or leave-on hair and scalp care compositions such as shampoos, conditioners, creams, lotions, gels, serums, mousses or oils. Rinse-off hair and scalp care compositions such as shampoos and conditioners are preferred.

The piroctone olamine will generally be included in a composition for use in the invention at a level of from 0.05 to 5%, more preferably from 0.1 to 2%, most preferably from 0.2 to 1% (by weight based on the total weight of the composition).

Compositions for use in the invention will generally include a cosmetically acceptable vehicle. The term "cosmetically acceptable" means that the vehicle is suitable for topical application to the skin, has good aesthetic properties, is compatible with the at least one biotin binding compound and the at least one precursor for biotin biosynthesis selected from pimelic acid and/or salts thereof and any other ingredients, and will not cause any safety or toxicity concerns.

The vehicle may comprise an aqueous phase, an oil phase, an alcohol, a silicone phase or a mixture thereof, and may be in the form of an emulsion. Emulsions can have a range of consistencies including thin lotions (which may also be suitable for spray or aerosol delivery), creamy lotions, light creams and heavy creams.

Compositions for use in the invention may also be formulated in a single-phase carrier such as a hydrophobic or hydrophilic liquid. Suitable hydrophobic liquid carriers include liquid polyorganosiloxanes, mineral oils, hydrogenated polyisobutene, polydecene, paraffins and isoparaffins of at least 10 carbon atoms, aliphatic or aromatic ester oils (such as isopropyl myristate, lauryl myristate, isopropyl palmitate, diisopropyl sebacate, diisopropyl adipate and C₁₂ to C₁₅ alkyl benzoates), polyglycol ethers (such as polyglycol butanol ethers) and mixtures thereof. Suitable hydrophilic liquid carriers include water, monohydric or polyhydric aliphatic alcohols having 2 to 8, preferably 2 or 3 carbon atoms (such as ethanol and isopropanol, oligoglycol ethers having 2 to 5 repeat units (such as dipropylene glycol) and mixtures thereof.

Liquid form compositions for use in the invention may be thickened, for example using one or more water soluble or colloidally water soluble polymeric thickening agents. Suitable water soluble or colloidally water soluble polymeric thickening agents include hydroxyethyl cellulose, methyl cellulose, hydroxypropyl methyl cellulose, polyquaternium-10, carrageenan, guar gum, hydroxypropyl guar gum, xanthan gum, polyvinylalcohol, acrylic acid/ethyl acrylate copolymers, carboxyvinyl polymers, cross-linked polyacrylate polymers and polyacrylamide polymers.

Preferred types of composition for use in the invention include shampoos, oils and lotions, which are intended for topical application to the hair and scalp.

Shampoo compositions for use in the invention are generally aqueous (i.e. they have water or an aqueous solution as their major component), and will suitably comprise from 50 to 98%, preferably from 60 to 90% water (by weight based on the total weight of the composition).

Shampoo compositions for use in the invention will typically comprise one or anionic surfactants such as sodium oleyl succinate, ammonium lauryl sulfosuccinate, ammonium lauryl sulphate, sodium dodecylbenzene sulphonate, triethanolamine dodecylbenzene sulfonate, sodium cocoyl isethionate, sodium lauryl isethionate, sodium N-lauryl sarcosinate, sodium lauryl sulfate, sodium lauryl ether sulfate (n) EO, (where n ranges from 1 to 3), ammonium lauryl sulfate and ammonium lauryl ether sulfate (n) EO, (where n ranges from 1 to 3).

Mixtures of any of the above described materials may also be used.

The total amount of anionic surfactant in shampoo compositions for use in the invention generally ranges from 5 to 30%, preferably from 8 to 20% (by weight based on the total weight of the composition).

Shampoo compositions for use in the invention may also include co-surfactants such as nonionic surfactants, which can be included in an amount ranging from 0.5 to 8%, preferably from 2 to 5% (by weight based on the total weight of the composition) and/or amphoteric or zwitterionic surfactants, which can be included in an amount ranging from 0.5 to 8%, preferably from 1 to 4% (by weight based on the total weight of the composition). Representative nonionic surfactants include alkanolamides such as cocamide monoethanolamide and cocamide monoisopropanolamide; alkyl polyglucosides such as cocoglucoside and lauryl glucoside; and acyl glucamides such as cocoyl methyl glucamide.

Mixtures of any of the above described materials may also be used.

Shampoo compositions for use in the invention may also include one or more cationic polymers, which can be included in an amount ranging from 0.01 to 5%, preferably from 0.05 to 2% (by weight based on the total weight of the composition). Representative cationic polymers include cationic polysaccharide polymers such as cationic cellulose derivatives and cationic guar gum derivatives such as guar hydroxypropyltrimethylammonium chloride.

Shampoo compositions for use in the invention may also include one or more suspending agents, which can be included in an amount ranging from 0.05 to 5%, preferably from 0.1 to 3% (by weight based on the total weight of the composition). Representative suspending agents include polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives such as ethylene glycol distearate.

Hair oils and lotions for use in the invention typically have an oil phase containing at one or more cosmetically acceptable fatty materials which may be liquid or solid at room temperature (25°C). Lotions are typically aqueous emulsions having an aqueous phase in addition to the oil phase.

Suitable cosmetically acceptable fatty materials include naturally derived oils (such as sunflower oil, borage oil, soybean oil, castor oil, olive oil and almond oil); esters of monoalcohols or of polyols with monocarboxylic or polycarboxylic acids, at least one of the alcohols and/or acids comprising at least one hydrocarbon-based chain containing at least 6 carbon atoms (such as octyl palmitate, isopropyl myristate, isopropyl palmitate, isopropyl isostearate, hexyl laurate, isohexyl laurate, isohexyl palmitate, decyl oleate, isodecyl oleate, hexadecyl stearate, decyl stearate, dihexyldecyl adipate, lauryl lactate, myristyl lactate, cetyl lactate, oleyl stearate, oleyl oleate, oleyl myristate, lauryl acetate, cetyl propionate, isononyl isononanoate, propylene glycol dicaprate, diisopropyl adipate, dibutyl adipate, and oleyl adipate); ethers (such as dicapryl ether); fatty alcohols (such as cetyl alcohol, stearyl alcohol and behenyl alcohol); isoparaffins (such as isooctane, isododecane and isohexadecane); silicone oils (such as cyclomethicone, dimethicone and dimethiconol); hydrocarbon oils (such as mineral oil, petrolatum and polyisobutene); fatty acids containing from 8 to 30 carbon atoms, (such as stearic acid, lauric acid, palmitic acid and oleic acid); vegetable fats (such as cocoa butter, coconut oil, palm oil and shea butter); petroleum-based, natural and synthetic waxes (such as lanolin wax, beeswax, carnauba wax, candelilla wax, paraffin wax, lignite wax, microcrystalline waxes, ceresin, ozokerite, and polyethylene waxes); hydrogenated oils which are solid at 25° C (such as hydrogenated castor oil, hydrogenated jojoba oil, hydrogenated palm oil, hydrogenated tallow and hydrogenated coconut oil);and fatty esters that are solid at 25°C (such as C₂₀₋₄₀ alkyl stearate).

The aqueous phase of lotions for use in the invention may also include one or more organic liquids that are miscible with water at room temperature (25°C). Exemplary water-miscible organic liquids include monohydric and polyhydric alcohols and derivatives thereof such as C₂-C₆ alkanols (such as ethanol and isopropanol); C₂-C₁₀ glycols and polyols (such as glycerol, propylene glycol, butylene glycol, pentylene glycol, hexylene glycol, caprylyl glycol, dipropylene glycol, and diethylene glycol); C₃-C₁₆ glycol ethers (such as mono-, di-, or tripropylene glycol (C₁-C₄) alkyl ethers and mono-, di-, or triethylene glycol (C₁-C₄) alkyl ethers) and polyethylene glycol having 2 to 12 oxyethylene units.

Lotions for use in the invention may also include surface active ingredients, such as emulsifiers and solubilizers, to enable two or more immiscible components to be combined homogeneously and to help stabilize the composition. Emulsifiers that may be used to form O/W or W/O emulsions include sorbitan oleate, sorbitan sesquioleate, sorbitan isostearate, sorbitan trioleate, PEG-20 sorbitan isostearate, polyglyceryl-3-diisostearate, polyglycerol esters of oleic/isostearic acid, polyglyceryl-6 hexaricinolate, polyglyceryl-4-oleate, polyglyceryl-4 oleate/PEG-8 propylene glycol cocoate, polyglyceryl-2 dipolyhydroxystearate, PEG-30 dipolyhydroxystearate, oleamide DEA, TEA myristate, TEA stearate, magnesium stearate, sodium stearate, potassium laurate, potassium ricinoleate, sodium cocoate, sodium tallowate, potassium castorate, sodium oleate, cetyl phosphate, diethanolamine cetyl phosphate, potassium cetyl phosphate, sodium glyceryl oleate phosphate, dimethicone copolyol, cetyl dimethicone copolyol, octyldimethicone ethoxyglucoside copolyol, dimethicone copolyol crosspolymer and laurylmethicone copolyol.

Combinations of any of the above described materials or product forms may also be used.

Compositions for use in the invention (as described above) may include additional actives for improving the physical and/or aesthetic characteristics of the scalp and/or the hair. Examples include amino acids, vitamins, minerals and/or antioxidants, emollients, humectants, sunscreens, anti-irritants, exfoliating agents, botanical extracts (such as pomegranate, white birch, green tea, chamomile and licorice extracts) and mixtures thereof.

Compositions for use in the invention (as described above) may include additional functional ingredients for improving the physical and/or aesthetic characteristics of the composition per se. Examples include inorganic pigments (such as titanium oxide, zirconium oxide, cerium oxide, zinc oxide, iron oxide, chromium oxide, manganese violet, ultramarine blue, chromium hydrate and ferric blue); organic pigments (such as carbon black and the organic lakes of barium, strontium, calcium or aluminium); pearlescent agents (such as mica coated with titanium oxide and/or iron oxide); dyes, preservatives (such as disodium EDTA, benzyl alcohol, methylparaben, phenoxyethanol, propylparaben, ethylparaben, butylparaben and isobutylparaben); pH adjusters and fragrances (such as essential oils, flower oils, natural extracts from resins, gums, balsams, beans, mosses and other plants, as well as synthetic aromatic materials).

Mixtures of any of the above described materials may also be used.

### Packaging and Use

A composition for use in the invention (as described above) may be packaged in a suitable container to suit its viscosity and intended use by the consumer. For example, a liquid composition can be packaged in a bottle or tube, or in a container fitted with a pump suitable for finger operation, or in a propellant-driven aerosol device. Gel or cream compositions can be packaged in a non-deformable bottle or squeeze container, such as a tube or a lidded jar, or in an applicator having a dispensing head provided with at least one aperture through which the composition can be extruded under mild pressure.

The composition is suitably applied to the hair and scalp and massaged into the surface of the scalp. Generally, an amount corresponding to about 1 to 15 ml of the composition per application is applied uniformly over the area of treatment daily or at least once a week over a time interval of at least 7 (seven) days, more preferably at least 30 (thirty) days.

The invention will be further illustrated by the following, non-limiting Examples.

### EXAMPLES

### Method

Sixty female subjects were recruited for the study and classified as healthy (30 subjects) or dandruff sufferers (30 subjects) based on total weighted head score assessment. 4mm scalp biopsy samples from both subject groups were taken at two different timepoints: at baseline, and after 4 weeks of treatment with a shampoo containing 0.5 wt% piroctone olamine. Half of each biopsy was homogenised and subjected to RNA extraction, QuantSeq3'FWD library preparation (Lexogen), and RNA sequencing on NextSeq500 platform (Illumina). Raw and normalised RNA-seq data were subject to rigorous QC evaluation, all samples passed QC test and were included in subsequent analysis.

### Results

Statistical models were fitted to the data to identify differentially expressed genes in the dandruff group compared to the healthy group.

In the scalp biopsy samples taken at baseline, the specific genes shown in Table 1 were found to be significantly differentially expressed in the dandruff group, as compared to the healthy group.

**Table 1**

| Gene symbol | Protein | Dandruff vs healthy at baseline | |
|---|---|---|---|
| | | Fold Change | P value |
| *Adhesion Molecules* | | | |
| DSG4 | Desmoglein 4 | -1.45 | 0.08067* |

| *Proteases* | | | |
|---|---|---|---|
| ADAM8 | ADAM metallopeptidase domain 8 | 1.51 | 0.00006 |
| PRSS22 | Serine protease 22 | 1.37 | 0.00146 |
| KLK6 | Kallikrein 6 | 1.45 | 0.00026 |
| KLK8 | Kallikrein 8 | 1.90 | 0.00001 |
| KLK11 | Kallikrein 11 | 1.20 | 0.00357 |
| KLK13 | Kallikrein 13 | 2.22 | 0.00002 |
| *Not significant (cut off P<0.05) | | | |

As shown in Table 2, after 4 weeks of treatment with 0.5wt% piroctone olamine shampoo the differential expression of these genes is reversed, indicative of a shift towards healthy state.

**Table 2**

| Gene symbol | Protein | Dandruff vs healthy after treatment | |
|---|---|---|---|
| | | Fold Change | P value |
| *Adhesion Molecules* | | | |
| DSG4 | Desmoglein 4 | 1.54 | 0.04343 |

| *Proteases* | | | |
|---|---|---|---|
| ADAM8 | ADAM metallopeptidase domain 8 | -1.54 | 0.00579 |
| PRSS22 | Serine protease 22 | -1.35 | 0.00197 |
| KLK6 | Kallikrein 6 | -1.22 | 0.02658 |
| KLK8 | Kallikrein 8 | -1.60 | 0.00055 |
| KLK11 | Kallikrein 11 | -1.23 | 0.00088 |
| KLK13 | Kallikrein 13 | -2.15 | 0.00002 |

### Conclusions

The results in Table 1 show unfavourable alterations in the gene expression profile of cell adhesion molecules and proteases in the condition of dandruff.

The decrease in gene expression of desmosomal DSG4 and the increase in gene expression of several proteases may contribute to weakening of hair club fibre anchorage, thus favouring premature hair club fibre release.

However, Table 2 shows that the treatment with piroctone olamine in accordance with the invention reverses the unfavourable alterations

The increase in gene expression of desmosomal DSG4, and the decrease in gene expression of several proteases may restore stronger hair club fibre anchorage, thus inhibiting premature hair club fibre release.

This indicates that treatment with piroctone olamine can restore the cell adhesion molecule and protease gene expression profile towards one that resembles healthy signature, resulting in improved hair club fibre anchorage and consequently reduced shedding.

## Claims

1. Cosmetic use of piroctone olamine as an active ingredient for upregulating the expression of genes encoding cell adhesion molecules in cells of the hair follicle, and/or downregulating the expression of genes encoding proteases in cells of the hair follicle, thereby reducing shedding of hair club fibres from hair follicles.

2. Cosmetic use according to claim 1, in which the piroctone olamine is formulated into a rinse-off hair and scalp care composition.

3. Cosmetic use according to claim 1 or claim 2, in which the piroctone olamine is included in the rinse-off hair and scalp care composition at a level of from 0.05 to 5% (by weight based on the total weight of the composition).

4. Cosmetic use according to any one of claims 1 to 3, in which the genes encoding cell adhesion molecules are selected from genes encoding desmosomal cadherins.

5. Cosmetic use according to claim 4, in which the gene encoding cell adhesion molecules is DSG4.

6. Cosmetic use according to any one of claims 1 to 3, in which the genes encoding proteases are selected from genes encoding metalloproteases and serine proteases.

7. Cosmetic use according to claim 6, in which the genes encoding proteases are selected from any or all of ADAM8, PRSS22, KLK6, KLK8, KLK11 and KLK13.
